(19) **Europäisches Patentamt / European Patent Office / Office européen des brevets**

(11) **EP 2 106 748 A1**

(12) # EUROPEAN PATENT APPLICATION

(43) Date of publication:
**07.10.2009 Bulletin 2009/41**

(51) Int Cl.:
**A61B 5/107** (2006.01)   **A61B 1/04** (2006.01)

(21) Application number: **09156573.9**

(22) Date of filing: **30.03.2009**

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority: **31.03.2008 JP 2008092261**

(71) Applicant: **HOYA Corporation**
**Shinjuku-ku**
**Tokyo 161-8525 (JP)**

(72) Inventors:
- **Takahashi, Masao**
  **Tokyo Tokyo 161-8525 (JP)**
- **Yokoyama, Yuko**
  **Tokyo Tokyo 161-8525 (JP)**
- **Ikemoto, Yosuke**
  **Tokyo Tokyo 161-8525 (JP)**

(74) Representative: **Schaumburg, Thoenes, Thurn, Landskron, Eckert**
**Patentanwälte**
**Postfach 86 07 48**
**81634 München (DE)**

(54) **Endoscope measuring 3-D profile**

(57)    An endoscope system has a scanner, a projector, and a measurement processor. The scanner is configured to scan light that passes through an optical fiber over a target by directing the light emitted from the distal end of an endoscope. The projector is configured to project a pattern on the target by switching on and off the light during scanning. Then, the measurement processor acquires a three dimensional (3-D) profile of the target on the basis of the shape of the pattern projected on the target.

## FIG. 7

Printed by Jouve, 75001 PARIS (FR)

EP 2 106 748 A1

**Description**

BACKGROUND OF THE INVENTION

1.Field of the Invention

The present invention relates to an endoscope system, and in particular, it relates to a process for measuring a three-dimensional (3-D) profile of a target to be observed, such as biological tissue.

2.Description of the Related Art

An endoscope system with 3-D measurement function illuminates a target having a convex shape such as a cubic shape, and measures the 3-D profile of the target, or the size of the 3-D profile, on the basis of light reflected from the target. In Japanese unexamined publication JP1998-239030A, JP1998-239034A, JP1997-61132A, an endoscope system that measures a 3-D profile using a trigonometric method or a light-section method is described.
The trigonometric method calculates the height of the target from the displacement between an illumination position on an optical system and a light-receiving position on a photo-detector. On the other hand, the light-section method simultaneously projects a plurality of slit patterns by using a mask pattern, and calculates the height of the target from the distortion or deformation of the slit patterns.
When observing the interior walls of an organ, shape information of, say, a tumor (e.g., the dimensions of a polyp) is displayed on a monitor and is an important guidepost for a diagnosis. Therefore, in order for tissue to be discovered using an endoscope during an operation, the shape or size of the tissue must be acquirable in real time. Especially, the extent of the projection of the tissue from an inner wall of an organ is important in diagnosing the tissue. However, the process of calculating a 3-D profile takes time since a conventional endoscope with 3-D measurement function is designed such that the whole of the 3-D profile is precisely measured. Also, a conventional endoscope system with 3-D measurement function is equipped with an exclusive component such as a pattern mask, which is hard to use with a general-purpose endoscope without the 3-D measurement function.

SUMMARY OF THE INVENTION

An object of the present invention is to provide an endoscope system of simple construction that is capable of projecting a pattern freely and measuring the 3-D profile of a target instantaneously.
An endoscope system according to the present invention is capable of measuring a 3-D shape or profile of a target such as a portion of tissue using a simple construction and acquiring the useful 3-D profile instantaneously during operation of the endoscope.
The endoscope system has a scanner, a projector, and a measurement processor. The scanner is configured to scan light that passes through an optical fiber, onto the target, by directing the light exiting from the distal end of the endoscope. The optical fiber (scanning optical fiber) that delivers light for scanning is different from a light guide that directs light for illuminating the whole of a target and forming a target image. For example, a single or double cladding type, ultra thin optical fiber may be used. The scanner scans the light over the target in sequence.
The projector is configured to project a pattern on the target by switching the light on and off during the scanning. Thus, light for forming a pattern is cast on the target. Then, the measurement processor acquires a three dimensional (3-D) profile of the target on the basis of the shape of the pattern projected onto the target. Since the target has a 3-D profile, the pattern projected on the target is different from a pattern (the standard pattern) projected on a plane, namely, the projected pattern changes or deforms relative to the standard pattern. The measurement processor may obtain the 3-D profile from the shape of the projected pattern through various measuring methods.
In the present invention, various patterns are projectable on the target since the pattern is formed by turning the illumination light on or off in accordance with the scanning position. The precision of the recognized shape information varies with the shape or type of projected pattern. When the type or shape of the pattern is determined in accordance with the precision of the desired 3-D information, the operation time for calculating the 3-D information may sometimes be saved. For example, when finding only the size or height of the portion of tissue, the exact 3-D profile is not required. Therefore, the 3-D information may be obtained adequately and instantaneously by projecting a pattern that is sufficient for a diagnosis and for which it is easy to calculate the 3-D information. Considering that the operator may wish to select a pattern appropriate for the tissue, a selector for selecting a pattern from a plurality of patterns may be provided.
The projector may project a simple pattern, for example, a plurality of illumination spots on the target. Namely, an illumination spot having a size smaller than that of the target may be projected on the target by strobing the light. Since various deformations of the shape are detected from the projected illumination spots, a complicated 3-D profile is also adequately calculated from the shape of each illumination spot. Also, when gradient or height information of a target is required to calculate the 3-D profile, the projector may scatter a plurality of illumination spots on the target in the radial direction. This reduces calculation time since the height information can be obtained easily and adequately.

The scanner may scan the light over the target spirally. In this case, various patterns may be formed by adjusting the illumination timing of light. Namely, the size of the illumination spot or the intervals between neighboring spots may be freely adjusted in the radial or circumferential directions. When the optical fiber is a scanning optical fiber, the scanner may vibrate the tip portion of the scanning optical fiber in two dimensions. This allows the small illumination spots to be scattered on the target.

When projecting a pattern during an endoscopic operation, the illumination spot may be projected on the target while visible light for displaying an observed image is shone on the target. For example, a normal light source configured to emit visible light is provided. The emitted light is different from the light passing through the above optical fiber. The measurement processor may detect a pattern signal corresponding to the pattern from image signals that are read from an image sensor provided in the distal end of the endoscope. For example, the measurement processor detects the pattern signal on the basis of luminance signals or color signals found in the image signals. In order to separate the pattern signal from the image signals easily, the light may be chosen to be in a narrow wavelength band. For example, light having a specific narrow wavelength may be emitted.

In order to acquire the 3-D profile freely and at any time, a scanning scope may be provided and used with a conventional general-purpose endoscope system. The scanning scope has the optical fiber and the scanner, and is removably inserted into the endoscope. For example, the optical fiber may be provided within the endoscope, and the scanner may be provided in the distal end of the endoscope.

As for the projection of a pattern, the projector projects the pattern by selectively turning the light on/off while controlling the drive of a light source for a scanning. On the other hand, a spatial light modulation device may be provided in the distal end of the endoscope. In this case, the projector projects the pattern by selectively strobing the spatial light modulation device.

The measurement processor may measure the 3-D profile of the target from the degree of deformation of the pattern relative to a standard pattern obtained by projecting the pattern on a plane. When projecting a plurality of illumination spots as a pattern, the measurement processor finds the gradient of each spot by defining a right triangle with a hypotenuse that corresponds to the diameter of a standard circular illumination spot and a base that corresponds to the diameter of the main axis of the deformed illumination spot. Then, the measurement processor obtains the height of each illumination spot in turn, by finding the relative height between neighboring illumination spots.

The measurement processor may calculate the size of the 3-D profile. In this case, the projector projects a plurality of illumination spots along the circumference of the 3-D profile of the target to emphasize the area of the 3-D portion of the target. The measurement processor may recognize the 3-D profile of the target by using the contour image or the TIN (Triangulated Irregular Network) . When using the TIN, the measurement processor may select two end points of a line that intersects a gradient direction and has relatively large vertical angles.

An apparatus for projecting a pattern according to another aspect of the present invention has a scanning controller that controls a scanner, and a projector configured to project a plurality of illumination spots on the target in accordance with a scanning position by switching the light on and off during the scanning. The scanning controller may control the scanner so as to scan light when a projection process or a measurement process is initiated. The scanner is configured to scan light that passes through an optical fiber over a target by directing the light from the distal end of an endoscope. For example, a scanning scope with the scanner and the optical fiber is connectable to the apparatus.

An apparatus for measuring a 3-D profile of a target according to another aspect of the present invention has a signal detector that detects signals corresponding to the illumination spots; and a measurement processor that acquires the 3-D profile of the target from the degree of deformation of the illumination spots relative to a standard illumination spot obtained by projecting the pattern on a plane. The measurement processor finds the gradient of each illumination spot from the degree of deformation of the illumination spots, and obtains height information of the 3-D profile from the series of calculated gradients.

A computer-readable medium that stores a program for projecting a pattern according to another aspect of the present invention has a scanning control code segment that controls a scanner, the scanner configured to scan light that passes through an optical fiber over a target by deflecting the light which exits from the distal end of an endoscope; and a projection code segment that switches the light on and off during the scanning to project a plurality of illumination spots in accordance with a scanning position.

A computer-readable medium that stores a program for measuring the 3-D profile of a target according to another aspect of the present invention has a signal detection code segment that samples signals corresponding to the illumination spots; and a measuring process code segment that acquires the 3-D profile of the target from the degree of deformation of the illumination spots relative to a standard illumination spot obtained by projecting the pattern on a plane. The measuring process code segment finds the gradient of each illumination spot from the degree of deformation of the illumination spots. The measuring process code segment obtains height information of the 3-D profile from the series of calculated gradients.

A method for projecting a pattern according to another aspect of the present invention includes: a) scanning light that passes through an optical fiber over a target by deflecting the light which exits from the distal end of an endoscope; b) controlling the scanner; and c) projecting a plurality of illumination spots on the target in accordance with the scanning position by switching the light on and off during the scanning.

A method for measuring the 3-D profile of a target according to another aspect of the present invention includes: a) detecting signals corresponding to the illumination spots described in Claim 26; and b) acquiring the 3-D profile of the target from the degree of deformation of the illumination spots relative to a standard illumination spot obtained by projecting the pattern on a plane. The method further includes: d) finding the gradient of each illumination spot from the degree of deformation of the illumination spots; and e) the measurement processor obtaining height information on the 3-D profile from the series of calculated gradients.

BRIEF DESCRIPTION OF THE DRAWINGS

The present invention will be better understood from the description of the preferred embodiments of the invention set forth below together with the accompanying drawings, in which:

Fig. 1 is a block diagram of an endoscope system according to a first embodiment;
Fig. 2 illustrates a schematic scanning optical fiber and scanning unit;
Fig. 3 shows the main flowchart for the measurement process that measures 3-D profile information of a target;
Fig. 4 is a subroutine of Step S102 shown in Fig. 3;
Fig. 5 illustrates a projected pattern image; and
Fig. 6 is a subroutine of Step S103 shown in Fig. 3;
Fig. 7 illustrates a pattern image projected on the target;
Fig. 8 illustrates a relationship between the deformation of an illumination spot and the gradient of the target;
Fig. 9 illustrates gradient characteristics between neighboring illumination spots;
Fig. 10 illustrates a contour image of the tissue;
Fig. 11 illustrates the 3-D shape of a tissue represented by using the TIN;
Fig. 12 illustrates the selection method of a remaining point;
Fig. 13 is a block diagram according to the third embodiment;
Fig. 14 is a block diagram of an endoscope system according to the fourth embodiment.
Figs. 15A to 15C illustrate a modification of a projected pattern different from the pattern shown in the first to fourth embodiments; and
Fig. 16 illustrates a projected pattern.

DESCRIPTION OF THE PREFERRED EMBODIMENTS

Hereinafter, the preferred embodiments of the present invention are described with reference to the attached drawings.

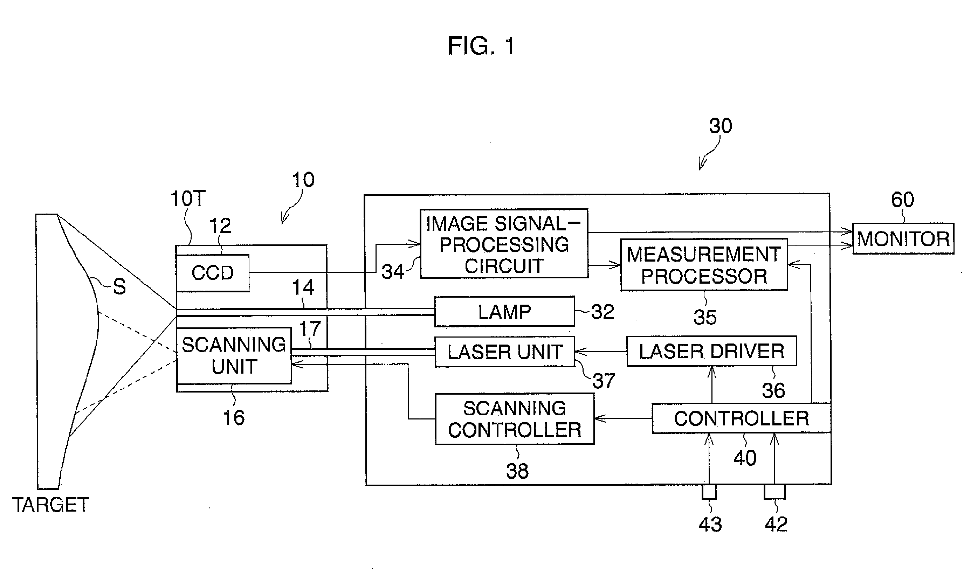

Fig. 1 is a block diagram of an endoscope system according to the first embodiment.

The endoscope system is equipped with a videoscope 10 having a CCD 12 and a processor 30. The endoscope 10 is detachably connected to the processor 40, and a monitor 60 and a keyboard (not shown) are also connected to the processor 40. An observation lamp 32, provided in the video processor 20, emits white light. The emitted light is delivered to the distal end 10T of the videoscope 10 by a light guide 14 composed of optic-fiber bundles. Light passing through the light guide 14 is cast from the distal end 10T of the videoscope 10 toward a target S.

Light reflected from the target S passes through an objective lens (not shown) and reaches the CCD 12, so that a subject image is formed on a light-receiving area of the CCD 12. On the light-receiving area of the CCD 12, a complementary color filter (not shown), checkered with a repeating pattern of the four color elements, Yellow (Y), Magenta (Mg), Cyan (Cy), and Green (G), is arranged such that each area color element is opposite a pixel. In the CCD 54, image-pixel signals are generated by the photoelectric effect based on light passing through the complementary color filters.

The generated analog image-pixel signals are read from the CCD 12 at regular time intervals (e.g., 1/60 or 1/50 second intervals). The read image-pixel signals are fed to an initial signal-processing circuit (not shown), in which an amplifying process, an A/D conversion process, and others are performed on the image-pixel signals to generate digital image signals. The generated digital image signals are fed to a signal-processing circuit 34 in the video processor 20. In the signal-processing circuit 34, various processes, including a white-balance process and a gamma-correction process, are performed on the image-pixel signals, so that R, G, and B video signals and luminance and color signals are generated. The video signals are directly output to the monitor 60. Thus, a full-color moving image is displayed on the monitor 60. A measurement processor 35 calculates three-dimensional (3-D) shape information for the target S.

A system controller 40 including a ROM unit, a RAM unit, and a CPU, controls the action of the videoscope 10 and the video processor 20 by outputting control signals to a laser driver 36 a scanning controller 38, and other circuit (s). A computer program associated with the control of the endoscope system is stored in the ROM.

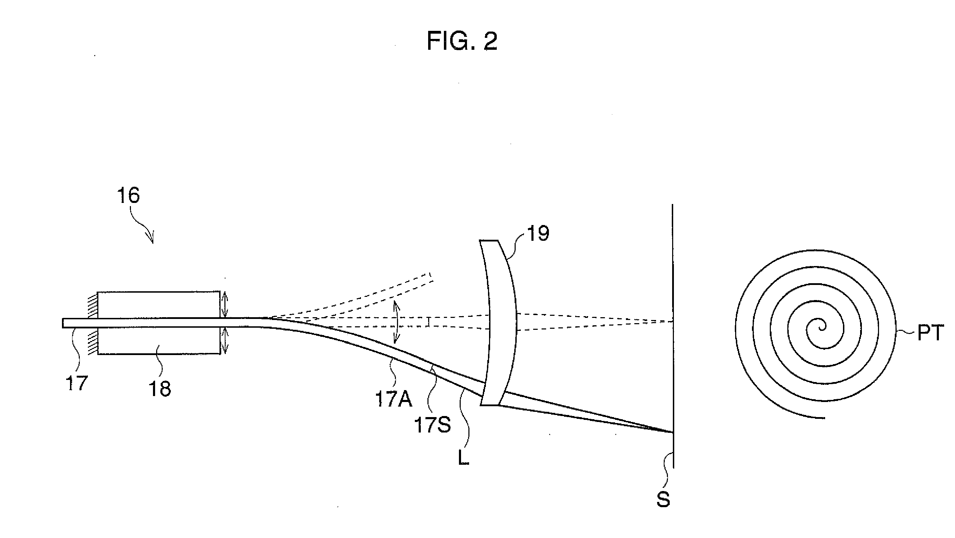

A single-type scanning optical fiber 17, different from the light guide 14, extends through the videoscope 10. A laser unit 37 provided in the video processor 20 emits narrow-band light of narrow-band wavelengths. The emitted narrow-band light is directed to the distal end 10T of the videoscope 10 by the optical fiber 17. As shown in Fig. 2, a scanning unit 16 is provided in the distal end 10T of the videoscope 10. The scanning unit 16 has a cylindrical actuator 18 and scans the narrow-band light over the target S. The optical fiber 17 passes along the axis of the actuator 18 and is supported thereby. A tip portion 17A of the scanning optical fiber 17 cantilevers from the actuator 18.

The actuator 18 fixed at the distal end 10T of the videoscope 10 a piezoelectric tubular actuator which vibrates the tip portion 17A of the optical fiber 17 two-dimensionally. Concretely, the actuator vibrates the tip portion 17A with respect to two axes perpendicular to each other, in accordance with a resonant mode. Since the tip portion 17A of the optical fiber 17 is a fixed-free cantilever, the vibration of the tip portion 17A displaces the position of the end surface 17S of the optical fiber 17 from the axis of the optical fiber 17.

The narrow-band light L emitted from the end surface 17S of the optical fiber 17 passes through an objective lens 19, and reaches the target S. As a result, the light emitted from the optical fiber 17, namely, the distal end 10T of the videoscope 10 is scanned by the vibration of the fiber tip portion 17A. The actuator 18 repeatedly or periodically vibrates the tip portion 17A so as to amplify the vibration along the two axes at a given time-interval. Consequently, the trace of the scanning beam, i.e., a scan line PT is formed in a spiral (see Fig. 2). The pitch of the spiral in the radial direction may be predetermined in accordance with the size of the target area.

Projection button 43 is a button for selecting a projecting pattern. Measurement button 42 is a button for initiating a measurement process. During a diagnosis, the measurement button 42 is operated to initiate acquisition of target shape information of the target displayed on monitor 60. Before initiating the measurement process, the operator selects a pattern from a set of patterns in accordance with the form of diagnosis or the organ to be observed. Herein, one of two patterns constructed of a plurality of illumination spots may be selected. One is a relatively short spot space between neighboring illumination spots; the other is a relatively long spot interval. During the scanning, image signals corresponding to the scanning beam are fed to the measurement processor 35, in which three-dimensional (3-D) profile information is acquired.

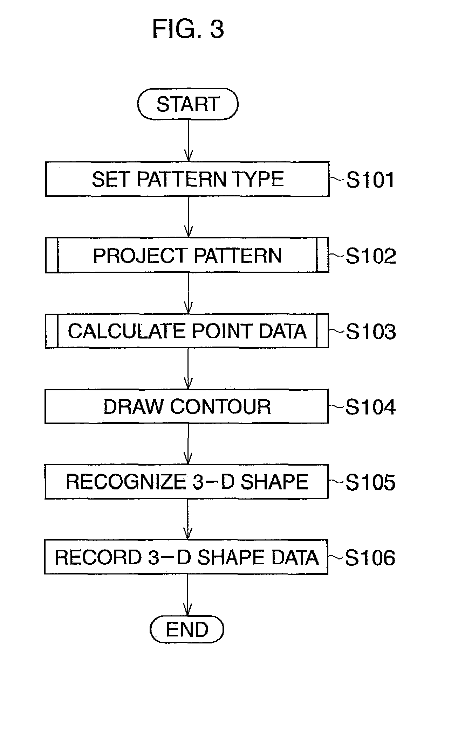

Fig. 3 shows a main flowchart of a measurement process that measures 3-D profile information of a target. Until the measurement button 42 is operated, the laser is not emitted but rather, a normal full-color image is displayed on the monitor 60. When the measurement button 42 is operated, the measurement process begins.

In Step S101, the pattern to be projected on the target, which is selected by the projection button 43, is set. In Step S102, the laser unit 37 emits narrow-band light (e.g., shortwave light such as blue light), and the scanning unit 16 vibrates the tip portion 17A of the scanning optical fiber 17 so as to draw a spiral trace on the target.

The laser driver 36 drives the laser unit 37 and controls the emission of the scanning beam, i.e., turns the beam ON or OFF at a given timing. The controller 40 synchronizes the scanning timing of the fiber tip portion 17A with the emission timing of the laser unit 37 by controlling the scanning controller 38 and the laser driver 36. The illumination or non-illumination is determined in accordance with a position of the fiber tip surface 17S displaced by the vibration, i.e., the scanning position of the scanning beam and a selected pattern.

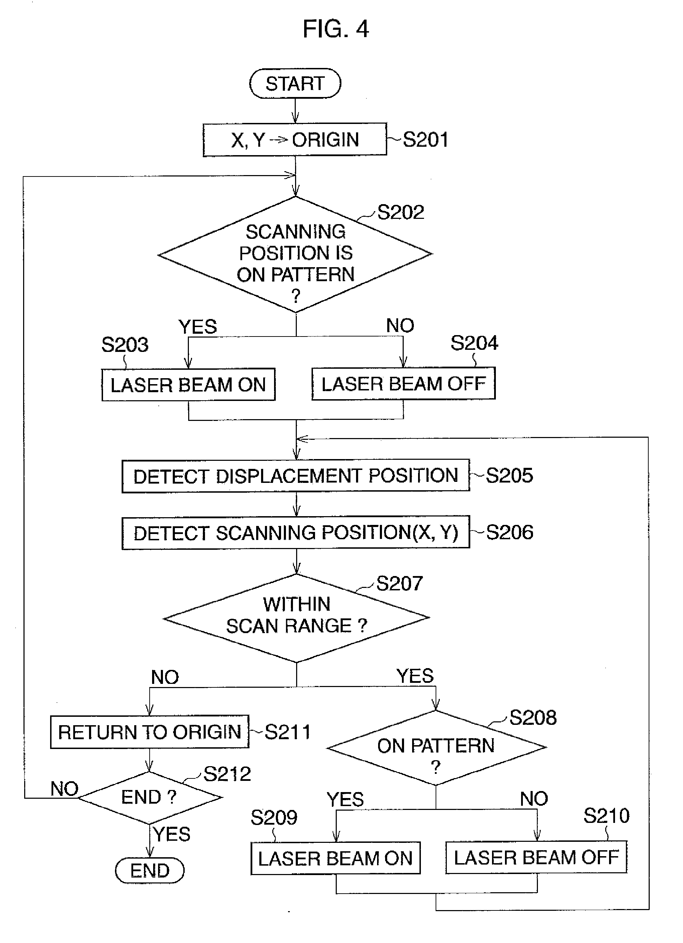

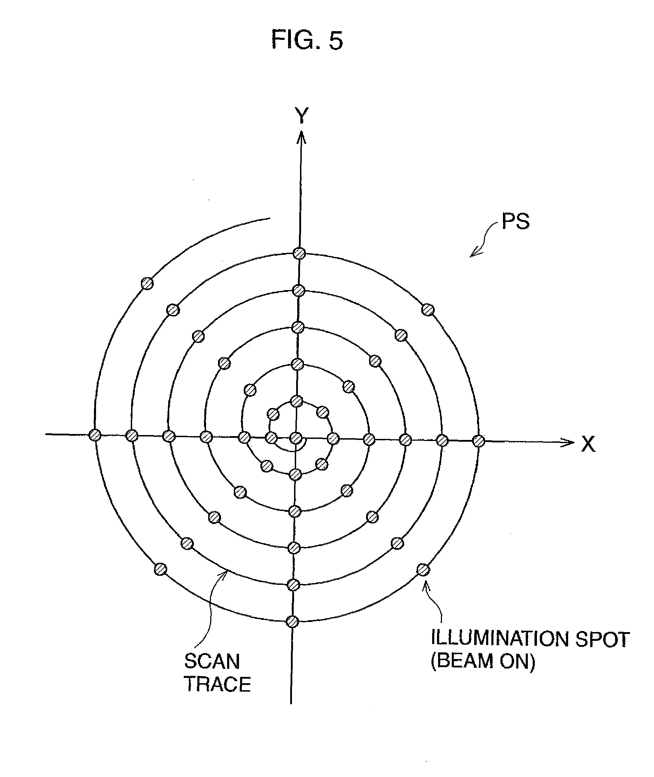

Fig. 4 is a subroutine of Step S102 shown inFig. 3. Fig. 5 illustrates a projected pattern image. The projection process is hereinafter explained with reference to Figs. 4 and 5.

The X-Y coordinates are herein defined on a projection area on an inner wall of an organ which includes the target area. The target area is perpendicular to the axis of the non-vibrating tip portion 17A of the scanning optical fiber 17. The position of the illumination spot is represented in X-Y coordinates. In Step S201, the position of the illumination spot is set to the origin (0, 0). Note that the intersection of the axis of the scanning fiber tip portion 17A and the projection area is defined as the origin (0, 0) .

The position of the illumination spot projected on the target area is uniquely decided by the displacement of the fiber tip surface 17S from the axis of the fiber tip portion 17A. Therefore, when driving the fiber tip portion 17A spirally, the position of the illumination spot can be detected on the basis of the frequency of scanning and the amplitude of the fiber tip portion 17A during the vibration.

As shown in Fig. 5, the projected pattern is a radial pattern SP, in which a plurality of illumination spots is scattered in the radial direction. The illumination spots are scattered such that they fall on straight lines which pass through the origin and are separated by 45-degree intervals. The size of each illumination spot depends upon the diameter of the fiber tip portion 17A and the optical lens 19, etc. The diameter of the optical fiber 17 or the power of the lens 19 is adjusted in accordance with the required precision of the measurement value, i.e., the 3-D profile data. Also, the size of each illumination spot may be set such that the illumination spots are interspersed on a three-dimensional target with spaces in between.

At Step S202 in Fig. 4, it is determined whether the current scanning position corresponds to a position on the pattern PS. When it is determined that the scanning position is a position on the pattern PS to be projected, the laser beam is emitted so as to illuminate narrow narrow-band light over the target S (S203). On the other hand, when it is determined that the scanning position is out of the pattern, the laser beam is turned off (S204).

As the fiber tip surface 17 moves in spiral displacement direction, the position of the scanning position also moves in a spiral scanning direction. In Step S205, the displacement position of the tip surface 17S of the optical fiber 17 is detected, and in Step S206, the coordinates of the current scanning position is detected. In Step S207, it is determined whether the scanning position is within the scan range. When it is determined that the scanning position is within the scan range, the process goes to Step S208, in which it is determined whether the scanning position is on the pattern PS. When it is determined that the scanning position is on the pattern, the laser beam is illuminated (S209), whereas the laser beam is turned OFF when the scanning position is out of the pattern (S210).

On the other hand, if it is determined at Step S207 that the scanning position is out of the scanning range, the process returns to Step S202 (S211). The projection process continues until the digital image signals of the all illumination spots are obtained (S212). Instead, the projection process may continue until a given time passes. After the projection process is finished, the process goes to Step S103 in Fig. 3. In Step S103, the position and the height of each illumination spot is calculated to give point data.

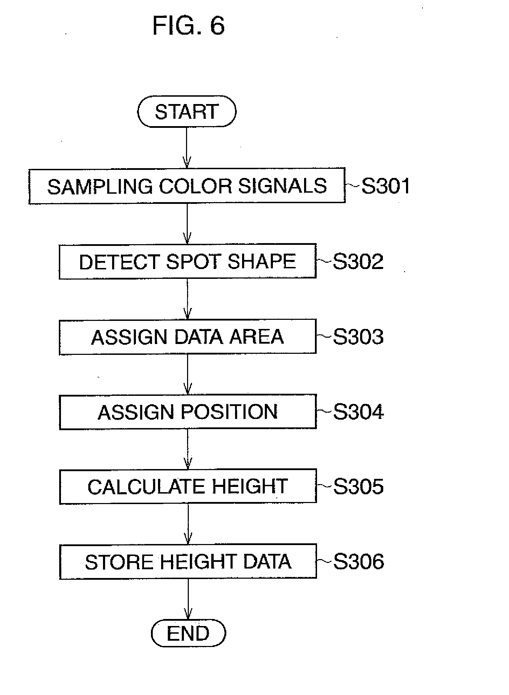

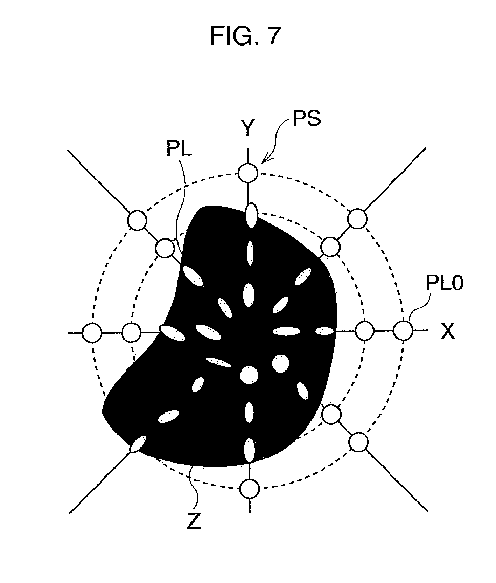

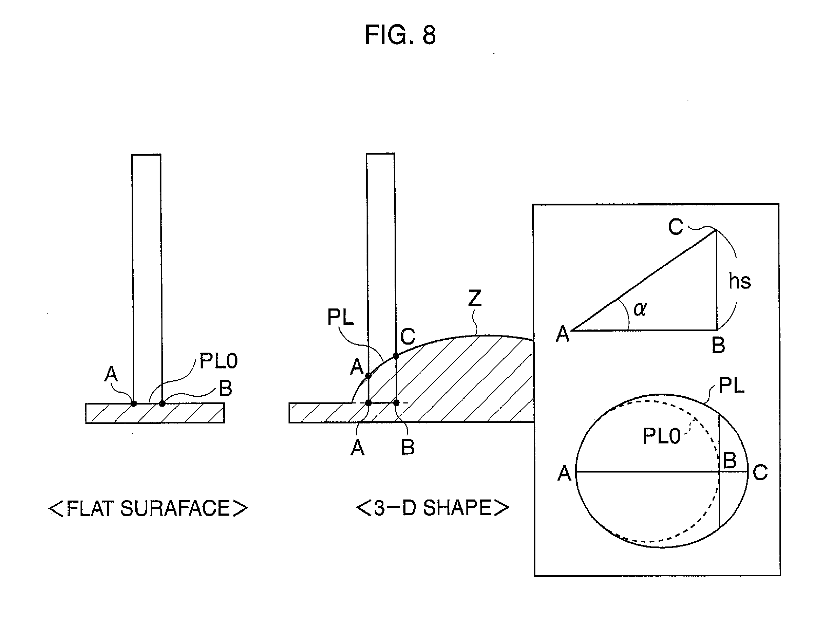

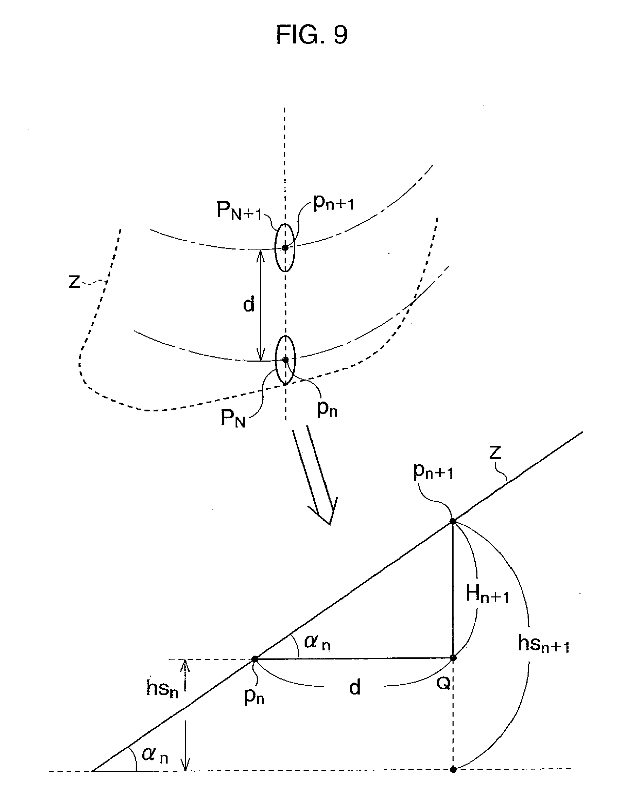

With reference to Figs. 6 to 9, the measurement of the 3-D profile of the target, i.e., the measurement of the height of each illumination spot is explained. Fig. 6 is a subroutine of Step S103 shown in Fig. 3. Fig. 7 illustrates a pattern image projected on the target. Fig. 8 illustrates the relationship between the deformation of an illumination spot and the gradient of the target. Fig. 9 illustrates gradient characteristics between neighboring illumination spots.

In Step S301, color signals are sampled from the digital image signals in the measurement processor 35. Since the laser unit 37 emits light of a narrow, shortwave band, a pattern image having specific colors (e.g., blue color) is included in the observed image. In Step S302, an outline shape of each illumination spot is detected from the color signals.

In Step S303, data area is assigned to each illumination spot, and in Step S304, position coordinates are assigned to each illumination spot. Herein, the center position of each illumination spot is regarded as a position coordinate. Then, in Step S305, the gradient and height of each illumination spot are calculated as explained below. Note that the height of the illumination spot indicates the height of the center position of the illumination spot.

As shown in Fig. 7, when pattern PS on an observation area including a tissue Z with a 3-D profile along a radial direction, the shape of projected spotlight deforms due to a gradient surface of the tissue Z. Specifically, while an illumination spot PL0 projected on a flat surface is formed in perfect circle, an illumination spot PL projected on a gradient surface of the tissue Z is forms an ellipse or oval figure (see Fig. 8). The illumination spot PLO is hereinafter defined as a standard illumination spot.

Comparing the illumination spot PL projected on the gradient surface with the standard illumination spot PL0, the following equation is obtained. Note that "α" represents a gradient of the position of the illumination spot PL, "hs" represents the height along vertical direction to the illumination spot PL. Also, "A" and "C" indicate end points along the major axis of an elliptical illumination spot PL, and "B" indicates the end point of circular illumination spot PLO, corresponding to the end point "C" of the illumination spot PL.

$$\alpha \;=\; \texttt{arccos(AB/AC)} \qquad\qquad\qquad (1)$$

$$\texttt{hs}^2 \;=\; \texttt{AC}^2 \;+\; \texttt{AB}^2 \qquad\qquad\qquad (2)$$

As shown in Fig. 8, when the pattern is projected on tissue Z from the vertical direction relative to an observation area KT including the tissue Z, it is regarded that the end point "C" and the end point "B" are on the same vertical line. Suppose that the right triangles having the vertices "A, B, and C" is defined, an interior angle "α" between the side "AB" and the side "AC" represents a gradient of the projected illumination spot PL. On the other hand, the length of the side "BC" represents the height of the end point "C" from the end point "A", namely, the height "hs" of the illumination spot PL.

The length of the main axis "AB" is decided by the size of the illumination spot PLO. Also, the length of the side "AC" is obtained by measuring the length of the main axis of the illumination spot PL. Therefore the gradient "α" and the height "hs" along the vertical direction of the illumination spot PL are calculated from equations (1) and (2).

In Fig. 9, one illumination spot and an adjacent illumination spot are shown. As for a series of illumination spots arrayed along a common gradient line, the main axis of each illumination spot generally faces the same gradient direction. Furthermore, if the degree of the deformation of an illumination spot is not greatly different from that of an adjacent illumination spot, the gradient of the surface between the neighboring illumination spots is supposed to be a constant (see Fig. 9). Therefore, when the gradient and height of an illumination spot at relatively low position is calculated, the height of a neighboring illumination spot can be obtained.

Concretely speaking, for example, the center point of an illumination spot $P_N$ projected onto a relatively low position is denoted "$p_n$", and the center point of an adjacent illumination spot $P_{N+1}$ in the same gradient direction and projected onto a relatively high position is denoted "$p_{n+1}$". When the gradient of the illumination spot $P_N$ is "$\alpha_n$" and the height of the illumination spot $P_N$ is "$hs_n$", it is regarded that the gradient between the illumination spot $P_N$ and the adjacent illumination spot $P_{N+1}$ is also "$\alpha_n$" (see Fig. 9) . Hence, when defining the right triangle having the hypotenuse "$p_n p_{n+1}$", the distance "$H_{n+1}$"along the vertical direction between the illumination spots $P_N$ and $P_{N+1}$ is obtained by the following equation. Note that "d" represents the length of the base of the right triangle.

$$H_{n+1}= d \times \tan\alpha_n \qquad\qquad (3)$$

The length "d" of the base represents a predetermined pitch between neighboring illumination spots, which is along the radial direction relative to the spiral scanning line. Therefore, when the gradient "$\alpha_n$" is calculated, the distance "$H_{n+1}$" is calculated by the equation (3). Then, the height "$hs_{n+1}$" of the illumination spot $P_{N+1}$ from the standard surface (flat surface), i.e., the height of the tissue Z at the illumination spot $P_{N+1}$, is calculated by adding the distance $H_{n+1}$ to the height $hs_n$ of the illumination spot $P_N$.

Thus, height information of each illumination spot can be calculated in order by calculating the gradient and the height of a series of illumination spots arrayed in a common gradient direction, from a lowest positioned illumination spot toward a highest positioned illumination spot. In the case of the radial pattern PS shown in Fig. 7, the gradient of the illumination spot and the height between the neighboring illumination spot is calculated in turn from the outward illumination spot toward a central illumination spot, along the radial direction. Consequently, the whole of the 3-D profile of tissue Z is detected and recognized.

After heights of all illumination spots are calculated, height data of each illumination spot is stored in a memory (Step S306 of Fig. 6). Each height point is recorded while associating each height data with corresponding position coordinates. When the height data is stored, the subroutine shown in Fig. 6 is terminated, and the process goes to Step S104 in Fig. 3. In Step S104, contour lines are generated by connecting the position coordinates of the illumination spots having the same height. Thus, a contour image representing 3-D shape of the tissue Z is obtained.

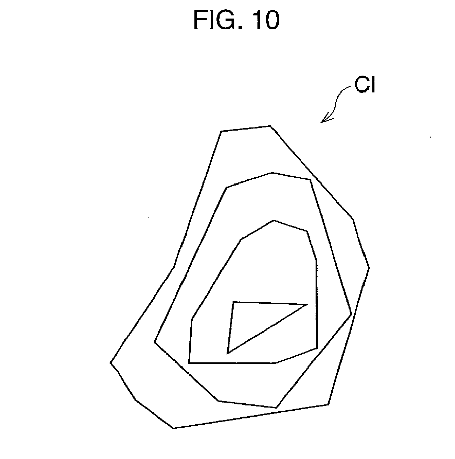

Fig. 10 illustrates a contour image CI of the tissue. The difference between a generated contour image and an actual image can be decreased by increasing the number of illumination spots and minimizing the size of the illumination spot. Also, an interpolation process such as bi-linear interpolation method may be used to make the contour lines smooth.

In Step S105, the contour image is transformed to a so-called "height image" so that a 3-D shape of the tissue is acquired. Furthermore, in addition to the 3-D shape information of the tissue, the size (diameter) of the tissue may be calculated on the basis of the 3-D shape information and displayed on the monitor 60, together with an observed image. In Step S106, 3-D information data is recorded in the RAM.

In this manner, when measuring the 3-D profile of the tissue, the scanning optical fiber 17 is utilized. The cantilevered tip portion 17A of the optical fiber 17 vibrates two-dimensionally by the actuator 18, so that the laser beam of specific wavelength, which is emitted from the laser unit 37, is repeatedly and periodically scanned in a spiral. During the scanning, the laser beam is selectively turned on/off in accordance with the scanning position. Thus, the plurality of illumination spots, which is scattered radially, is projected on the target area including the tissue.

When the color signals corresponding to the pattern are sampled from the digital image signals, the height and gradient of each illumination spot is calculated from the ellipse of the projected illumination spot. At that time, height data for each illumination spot is obtained by finding the series of gradients and the heights, in order, from an illumination spot on the lower position toward the illumination spot at the higher position. The contour image is calculated from each height point, and the height image is generated from the contour image.

Since the pattern is formed by selectively turning light on and off, various patterns can be made, and a scanning optical system is not required. Also, since the scanning line is spiral, the pitch of the illumination spots along the radial or circumferential directions may be adjusted to a given interval. Hence, the spot pattern may optionally be projected on a target in accordance with the size of the tissue or the precision of 3-D shape information required in the diagnosis. By increasing the precision of the measurement, the interval between neighboring illumination spots may be further reduced.

Furthermore, the process for obtaining the 3-D shape can be performed instantaneously since the 3-D shape information is acquired from the gradient of each illumination spot. Therefore, the operator can discern the size or shape of a portion of tissue in real time during the endoscope operation.

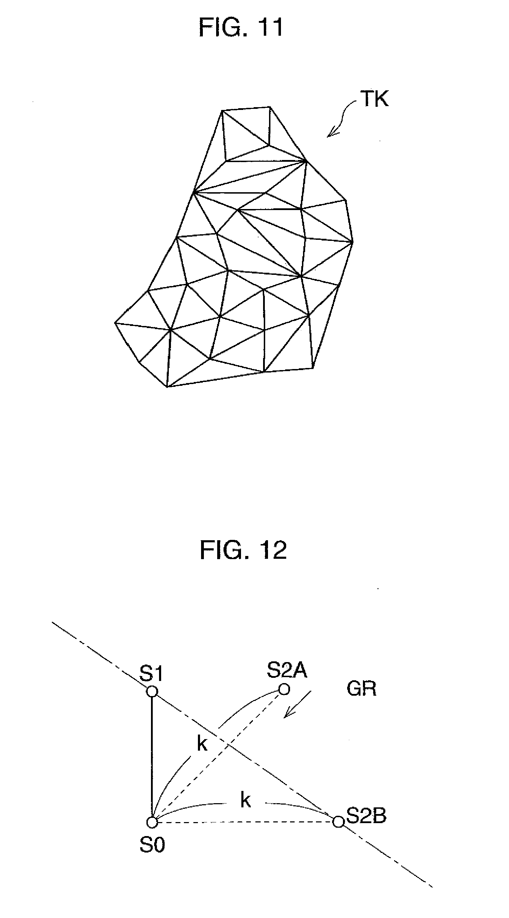

The second embodiment is explained with reference to Figs. 11 and 12. The second embodiment is different from the first embodiment in that the 3-D shape of a target is recognized by using the TIN (Triangular Irregular Network) instead of the drawing of contours. Other constructions are substantially the same as those of the first embodiment.

Fig. 11 illustrates a 3-D tissue structure represented by using the TIN. The TIN forms a 3-D surface shape by a set of non-overlapping irregular triangles. Herein, a triangle is defined by connecting point data (i.e., position data) for each illumination spot, which has height information, and the 3-D shape is represented by assembling a set of defined triangles.

When defining a triangle, one point is arbitrarily selected as a vertex, and two points which have a shorter interval than other points are selected. This selection is performed on each point and plural triangles are defined in order. Consequently, the series of triangles forms the TIN.

For example, suppose that a set of points is denoted by "P (=P1, P2, ..., Pn)", and a distance (Euclidean distance) between two points is denoted by "d= (p, q) ", a set of points pi(R(P, $p_i$)) is represented by the following equation.

$$R\ (P,\ p_i)$$

$$=\{p{\in}R2\,|\,d(p,\ p_i)<d(p,\ p_j)\ \text{for any}\ p_j{\in}P-\{p_i\}\}\qquad (4)$$

"$p_i$" makes a distance between the spot p and the set of points "P" shortest. R (P, $p_i$) (i=1, 2, ...,n) represents a straight line dividing a plane area.

In this embodiment, when selecting the last point for forming a triangle in the situation that two points have been selected as vertices and two points, which have the same interval with respect to one of the selected two points, exists, a point close to the line that intersects a gradient direction is selected as the remaining vertex.

Fig. 12 illustrates the selection method of the remaining point. Herein, the point S0 is a standard point for defining a triangle and a spot S1 has been selected as the vertex. Then, one of two points "S2A" and "S2B" is selected. The distance between the spot "S0" and a spot "S2A" is the same as the distance between the spot "S0" and a spot "S2B". In Fig.12, k represents the distance.

Comparing a triangle defined by the points "S0, S1, and S2A" with a triangle defined by the points "S0, S1, and S2B", the side "S0S2A" is along the gradient direction GR, whereas the side "S0S2b" is not along the gradient direction GR. Rather the side "S0S2B" intersects the gradient direction GR. The triangle defined by the points "S0, S1, and S2B" can take or incorporates detailed gradient information into a 3-D shape formed by the TIN and is able to reveal undulations in tissue, since more triangles can be arrayed along the gradient direction GR compared with the triangle defined by the points "S0, S1, and S2A". Hence, the point "S2B" is selected. Theses selections are performed in turn to acquire 3-D shape information on the tissue.

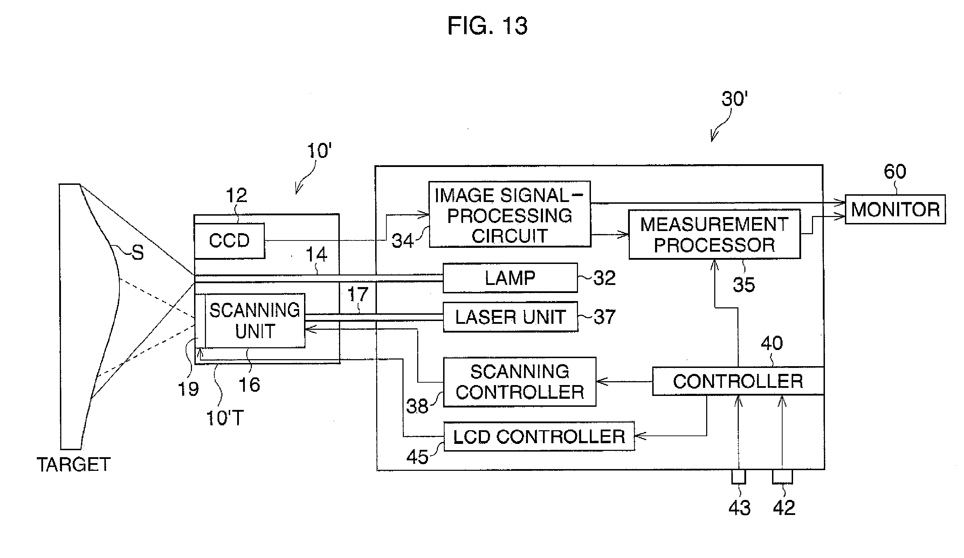

The third embodiment is explained with reference to Fig. 13. The third embodiment is different from the first embodiment in that an LCD shutter is used. Other constructions are substantially the same as those of the first embodiment.

Fig. 13 is a block diagram according to the third embodiment. In the distal end 10'T of a videoscope 10', an LCD shutter 19 composed of two-dimensionally arrayed LCDs is provided. The LCD shutter selectively passes or blocks light exiting from the optical fiber 17.

The controller 40 outputs control signals to an LCD controller 45 to synchronize the strobing of the LCD 19 with the scan timing of the scanning unit 16. The LCD 19 passes the emitted light when the scanning position is on a pattern, whereas the LCD 19 blocks the light when the scanning position is outside of the pattern. Note, any space modulation device (e.g., Digital Micro-mirror Device) other than the LCD shutter may optionally be used.

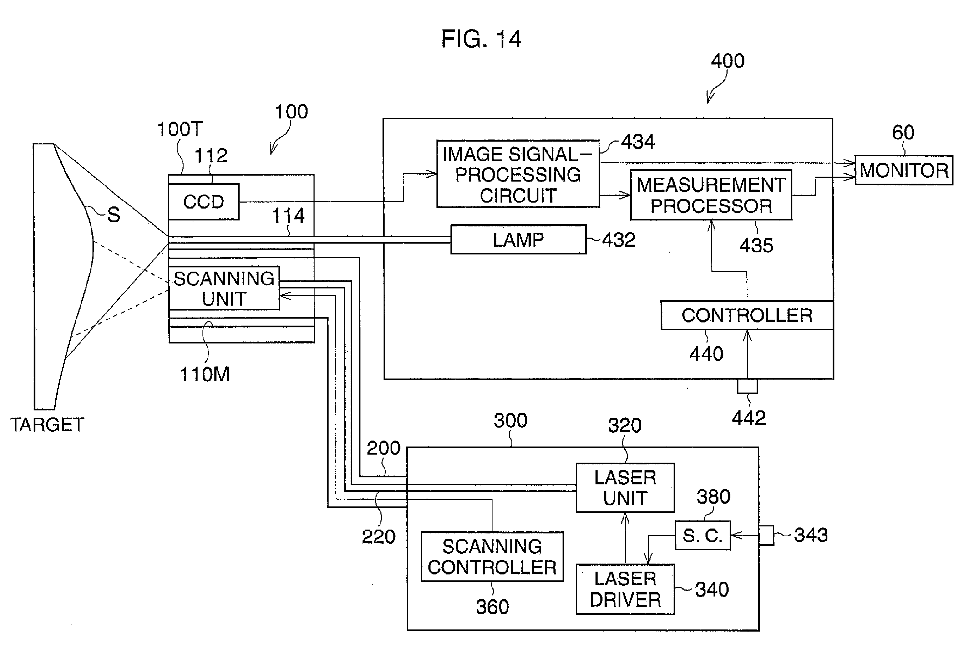

The fourth embodiment is explained with reference to Fig 14. The fourth embodiment is different from the first embodiment in that an independent scanning system and a projection system are included. Other constructions are substantially the same as those of the first embodiment.

Fig. 14 is a block diagram of an endoscope system according to the fourth embodiment.

In a videoscope 100 having a CCD 112 and a light guide 114, a scanning system is not provided. Instead, a probe type scanning scope 200 is used. The thin scanning scope 200 has a single type optical fiber 220, and is connected to a projection unit 300. A videoprocessor 400 is equipped with a lamp 432, an image signal-processing circuit 434, a measurement processor 435, and a controller 440.

The projection unit 300 is equipped with a laser unit 320, laser driver 340, a scanning controller 360, and a system controller 380. When measuring 3-D information of a tissue, the scanning scope 200 is inserted into a forceps channel 110M provided in the videoscope 100. The tip portion of the optical fiber 220 is driven spirally by a scanning unit 240 provided in the distal end of the scanning scope 200. The system controller 380 controls the emission of light from the laser unit 320 in accordance with a pattern selected by a pattern-selection button 343. The measurement process is performed in the video processor 400 by operating a measurement button 442. Note that an independent exclusive measurement unit may be provided.

As for the scanning, any construction that scans the light while deflecting the illumination of the light may optionally be used. For example, the light emitted from the scanning optical fiber may be scanned by changing the position of an optical system provided in the distal end of the videoscope. Also, an illumination unit, such as an LED, may be provided in the distal end of the videoscope. In this case, the illuminated light may be deflected in the unit. Furthermore, any two-dimensional sequential scan other than the above continuous spiral scan may optionally be applied. For example, a line scan may by used.

As for the pattern to be projected, the size of the illumination spot, and the radial or circumferential interval between neighboring illumination spots may be adjusted as required. Furthermore, other patterns other than the above radial scattered spot patterns may optionally be used.

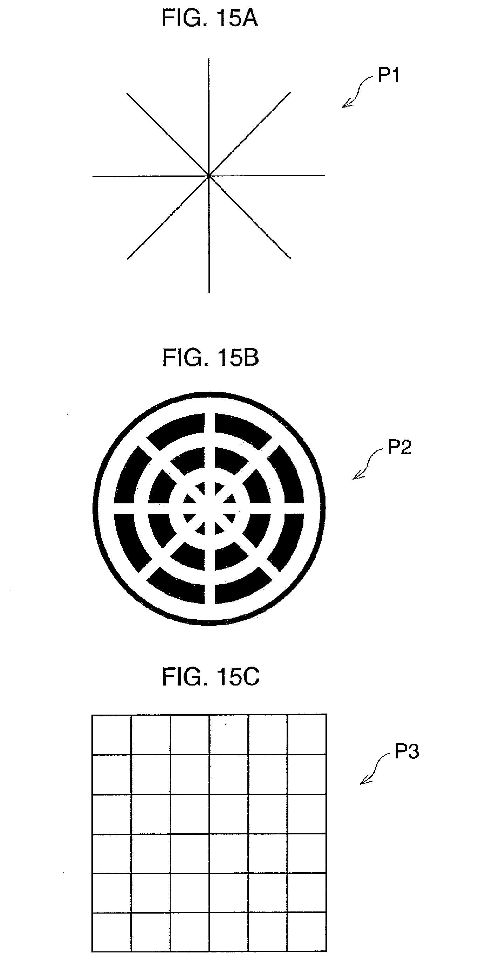

Figs. 15A to 15C illustrate a modification of a projected pattern different from the pattern shown in the first to fourth embodiments. In Fig. 15A, a radial line pattern is shown. In Fig. 15B, a concentric and radial pattern is shown. In Fig. 15C, a grid pattern is shown.

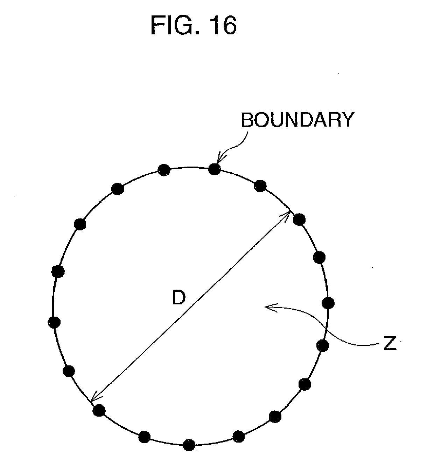

The fifth embodiment is explained with reference to Fig. 16. The fifth embodiment emphasizes a tissue boundary. Other constructions are the same as those of the first embodiment.

Fig. 16 illustrates a projected pattern. After a 3-D profile the tissue Z is measured, a boundary projecting process is performed. The size or diameter of the tissue Z is detected from a border of a non-deformed circular illumination spot and a deformed illumination spot. Namely, a boundary line is defined by drawing a line between circular illumination spots on a plane and the adjacent ellipse illumination spots on the cubic tissue, and the diameter is calculated on the basis of the boundary line.

The distal end of the videoscope faces the center of the tissue Z such that the origin coincides with the center of the tissue Z. Then, based on the calculated size of tissue Z, the tip portion of the optical fiber is driven spirally for scanning. When the scanning position is on the boundary of the tissue Z during the scanning, the laser beam is illuminated at a given interval. The boundary projection makes the boundary of the tissue distinctive so that the tissue is clearly recognized by an operator. Note that the size of the tissue may be measured in the first to fourth embodiments.

As for the shape information on the tissue, any shape characteristics other than the 3-D profile, or the size of the tissue, may be measured. Also, when obtaining only the size of the tissue, the recognition of the 3-D profile is not required. In this case, the size is obtained from a series of deformed illumination spots scattered along a circumference of the tissue. Any measurement method may optionally be used in accordance with a projected pattern when calculating a 3-D profile of a tissue.

**Claims**

1. An endoscope system comprising:

   a scanner configured to scan light that passes through an optical fiber over a target by directing the light emitted from the distal end of an endoscope;
   a projector configured to project a pattern on the target by switching the light on and off during the scanning; and
   a measurement processor that acquires a three dimensional (3-D) profile of the target on the basis of a shape of the pattern projected on the target.

2. The endoscope system of claim 1, wherein said projector projects a plurality of illumination spots on the target.

3. The endoscope system of claim 1 or 2, wherein said projector scatters a plurality of illumination spots on the target.

4. The endoscope system of any one of claims 1 to 3, wherein said projector scatters a plurality of illumination spots on the target in radial direction.

5. The endoscope system of any one of claims 1 to 4, wherein the scanner scans the light over the target spirally.

6. The endoscope system of any one of claims 1 to 5, wherein the optical fiber comprises a scanning optical fiber, said scanner vibrating the tip portion of the scanning optical fiber in two dimensions.

7. The endoscope system of any one of claims 1 to 6, further comprising a normal light source configured to emit visible light different from the light passing through the optical fiber, said measurement processor detecting a pattern signal corresponding to the pattern from image signals that are read from an image sensor provided in the distal end of the endoscope.

8. The endoscope system of any one of claims 1 to 7, wherein said measurement processor measures the 3-D profile of the target from a degree of deformation of the pattern relative to a standard pattern obtained by projecting the pattern on a plane.

9. The endoscope system of claim 8, wherein said projector projects a plurality of illumination spots on the target, said measurement processor calculating the gradient of each illumination spot from a degree of deformation of each illumination spot, said measurement processor calculating the 3-D profile on the basis of a series of calculated gradients.

10. The endoscope system of claim 9, wherein said measurement processor finds the gradient of each spot by defining a right triangle whose hypotenuse corresponds to the diameter of a standard circular illumination spot and whose base corresponds to the diameter of the main axis of a deformed illumination spot, said measurement processor obtaining the height of each illumination spot in turn, by finding the relative height between neighboring illumination spots.

11. The endoscope system of any one of claims 1 to 10, wherein a scanning scope that comprises the optical fiber and the scanner is removably inserted into the endoscope.

12. The endoscope system of any one of claims 1 to 11, further comprising a scanning light source configured to emit light, the light having a narrow wavelength band.

13. The endoscope system of any one of claims 1 to 12, further comprising a scanning light source configured to emit light, said projector projecting the pattern by selectively turning the light on and off while controlling the drive of said scanning light source.

14. The endoscope system of claim 1, wherein said measurement processor calculates the size of the 3-D profile.

15. An apparatus for projecting a pattern, comprising:

   a scanning controller that controls a scanner, said scanner configured to scan light that passes through an optical fiber over a target by directing the light emitted from the distal end of an endoscope; and
   a pro j ector configured to pro j ect a plurality of illumination spots on the target in accordance with a scanning position by switching the light on and off during the scanning.

16. An apparatus for measuring a 3-D profile of a target, comprising:

   a signal detector that detects signals corresponding to the illumination spots described in Claim 15; and
   a measurement processor that acquires the 3-D profile of the target from a degree of deformation of the illumination spots relative to a standard illumination spot obtained by projecting the pattern on a plane, said measurement processor finding the gradient of each illumination spot from the degree of deformation of the illumination spots, said measurement processor obtaining height information of the 3-D profile from the series of calculated gradients.

17. A method for projecting a pattern, comprising:

   scanning light that passes through an optical fiber over a target by directing the light emitted from the distal end of an endoscope;
   controlling the scanner; and
   projecting a plurality of illumination spots on the target in accordance with the scanning position by switching the light on and off during the scanning.

18. A method for measuring a 3-D profile of a target, comprising:

   detecting signals corresponding to the illumination spots described in Claim 17; and
   acquiring the 3-D profile of the target from the degree of deformation of the illumination spots relative to a standard illumination spot obtained by projecting the pattern on a plane, the method comprising determining gradient of each illumination spot based on the degree of deformation of the illumination spots, the method comprising obtaining height information of the 3-D profile from the series of calculated gradients.

## FIG. 1

EP 2 106 748 A1

FIG. 2

# FIG. 3

```
            ( START )
               |
               v
   +-------------------------+
   |    SET PATTERN TYPE     |--S101
   +-------------------------+
               |
               v
   +-------------------------+
   | |  PROJECT PATTERN    | |--S102
   +-------------------------+
               |
               v
   +-------------------------+
   | | CALCULATE POINT DATA| |--S103
   +-------------------------+
               |
               v
   +-------------------------+
   |     DRAW CONTOUR        |--S104
   +-------------------------+
               |
               v
   +-------------------------+
   |  RECOGNIZE 3-D SHAPE    |--S105
   +-------------------------+
               |
               v
   +-------------------------+
   | RECORD 3-D SHAPE DATA   |--S106
   +-------------------------+
               |
               v
            ( END )
```

# FIG. 4

START

X, Y → ORIGIN —S201

S202
SCANNING POSITION IS ON PATTERN ?

YES / NO

S203 LASER BEAM ON

S204 LASER BEAM OFF

DETECT DISPLACEMENT POSITION —S205

DETECT SCANNING POSITION(X, Y) —S206

S207
WITHIN SCAN RANGE ?

NO / YES

RETURN TO ORIGIN —S211

S212 END ?

NO

YES

END

S208
ON PATTERN ?

YES / NO

S209 LASER BEAM ON

S210 LASER BEAM OFF

## FIG. 5

SCAN TRACE

ILLUMINATION SPOT
(BEAM ON)

PS

# FIG. 6

```
        ( START )
            │
            ▼
┌─────────────────────────┐
│  SAMPLING COLOR SIGNALS  │─ S301
└─────────────────────────┘
            │
            ▼
┌─────────────────────────┐
│    DETECT SPOT SHAPE     │─ S302
└─────────────────────────┘
            │
            ▼
┌─────────────────────────┐
│    ASSIGN DATA AREA      │─ S303
└─────────────────────────┘
            │
            ▼
┌─────────────────────────┐
│     ASSIGN POSITION      │─ S304
└─────────────────────────┘
            │
            ▼
┌─────────────────────────┐
│    CALCULATE HEIGHT      │─ S305
└─────────────────────────┘
            │
            ▼
┌─────────────────────────┐
│    STORE HEIGHT DATA     │─ S306
└─────────────────────────┘
            │
            ▼
         ( END )
```

# FIG. 7

FIG. 8

<3-D SHAPE>

<FLAT SURAFACE>

# FIG. 9

FIG. 10

CI

# FIG. 11

# FIG. 12

**FIG. 13**

TARGET

S

CCD — 12

SCANNING UNIT

19  16  10'T  14  17

10'

34

IMAGE SIGNAL-PROCESSING CIRCUIT

LAMP — 32

LASER UNIT — 37

SCANNING CONTROLLER

38

LCD CONTROLLER

45

MEASUREMENT PROCESSOR

35

CONTROLLER — 40

43  42

MONITOR — 60

30'

EP 2 106 748 A1

# FIG. 14

EP 2 106 748 A1

## FIG. 15A

P1

## FIG. 15B

P2

## FIG. 15C

P3

FIG. 16

BOUNDARY

D

Z

Europäisches
Patentamt

European
Patent Office

Office européen
des brevets

**EUROPEAN SEARCH REPORT**

Application Number

EP 09 15 6573

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 5 436 655 A (HIYAMA KEIICHI [JP] ET AL) 25 July 1995 (1995-07-25) | 1-3,7,8, 11-13, 15-17 | INV.<br>A61B5/107 |
| Y | * column 7, lines 45-56 - column 11, lines 14-21; figures 1,4 * | 4-6 | ADD.<br>A61B1/04 |
| Y | US 5 103 497 A (HICKS JOHN W [US]) 7 April 1992 (1992-04-07) * column 3, lines 6-41; figures 2b,12 * | 4-6 | |

TECHNICAL FIELDS
SEARCHED      (IPC)

A61B
G06T

The present search report has been drawn up for all claims

| Place of search | Date of completion of the search | Examiner |
|---|---|---|
| Munich | 7 July 2009 | Fischer, Martin |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 09 15 6573

This annex lists the patent family members relating to the patent documents cited in the above-mentioned European search report.
The members are as contained in the European Patent Office EDP file on
The European Patent Office is in no way liable for these particulars which are merely given for the purpose of information.

07-07-2009

| Patent document cited in search report | | Publication date | Patent family member(s) | Publication date |
|---|---|---|---|---|
| US 5436655 | A | 25-07-1995 | NONE | |
| US 5103497 | A | 07-04-1992 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 10239030 A
- JP 10239034 A
- JP 9061132 A